# EUROPEAN PATENT APPLICATION

(11) **EP 1 130 099 A1**
(43) Date of publication of application: **05.09.2001**
(21) Application number: 01200711.8
(22) Date of filing: 26.02.2001
(51) Int. Cl.: C12N 15/13, C12N 15/12, C07K 16/18, A61K 39/395, G01N 33/68, G01N 33/577, A61P 35/00, C07K 14/78, C07K 19/00

(54) **Activated vitronectin as a marker of angiogenesis detected with phage antibodies**

(30) Priority: 25.02.2000 EP 00200660
(71) Applicant: Crucell Holland B.V., 2333 CN Leiden (NL)
(72) Inventor: Voest, Emile Eugene, 3768 AL Soest (NL); Bloemendal, Henri Johan, 3833 VN Leusden (NL); de Boer, Henriette Christine, 3704 LM Zeist (NL); Gebbink, Martijn Frans Ben Gerard, 3755 HL Eemnes (NL); Logtenberg, Ton, 3433 CH Nieuwegein (NL)
(74) Representative: Prins, Adrianus Willem

(57) **Abstract**

The present invention provides a method for producing antibody fragments that discriminate between native and activated vitronectin, which can be used as markers of angiogenesis. Also provided are antibodies and peptides comprising said fragments, and therapeutic methods, particularly related to targeting for antibody-mediated anti-vascular cancer therapy and inhibiting the adhesion and migration of tumor-endothelial cells and platelets.

## Description

### FIELD OF THE INVENTION

The invention relates generally to medicine and the use of antibodies. In particular the invention relates to antibody fragments (and derivatives thereof) which have a higher affinity for activated vitronectin than for native vitronectin. Said fragments can be used as markers of angiogenesis and other vitronectin-related diseases.

Antibodies are proteins that are produced by cells of the immune system, so-called B lymphocytes, in response to encounter with potentially harmful miro-organisms such as bacteria and viruses. Antibodies are capable of binding to the invading micro-organism that elicited their production and aid in their elimination. This activity of the immune system of producing antibodies in response to an invading ,micro-organism has been exploited in the production of monoclonal antibodies, a technology developed by Köhler and Milstein (1975). Monoclonal antibodies are all those immunoglobulin molecules that are produced by the progeny of a single B lymphocyte. Conventionally, monoclonal antibodies are obtained by immunizing a mouse with an antigen and fusing the spleen or lymphnode B lymphocytes with an immortalized murine plasma cell line. The ensuing hybrid cell lines (hybridomas) bear the characteristics of both parental cell types: they are immortal and produce a single species of monoclonal antibody specific for the antigen used to immunize the mouse. The advantage of monoclonal antibodies is that they represent a homogeneous population of immunoglobulin molecules with a pre-defined binding specificity. Over the years, monoclonal antibodies have proven invaluable tools in research and diagnostics.

Soon after the invention of hybridoma technology, the enormous potential of monoclonal antibodies in human therapy was realized. Because of their high binding specificity, antibodies were hypothesized, among others, to be capable of binding to viruses and bacteria and their toxic products facilitating their elimination. In other applications, monoclonal antibodies were envisaged to specifically bind to tumor cells to promote their eradication or to bind to soluble molecules produced by cells of the immune system to neutralize their activity in harmful chronic inflammatory conditions and/or in autoimmune disease. Indeed, monoclonal antibodies have been described as magic bullets that could be used in the treatment of a wide variety of human diseases.

Numerous clinical studies with monoclonal antibodies of non-human, usually murine origin showed that they performed poorly as a result of their immunogenicity; upon injection of murine antibodies in humans, the human immune systems recognizes the murine antibodies as foreign proteins resulting in the induction of an immune response against the murine protein. In addition, murine antibodies have poor pharmacokinetic properties in humans and are inefficient in recruiting effector functions of cells of the immune system. These issues have spurred the development of alternative strategies to obtain more human antibodies for therapy.

In one approach of creating more human antibodies, the immunoglobulin variable regions encoding murine antibodies are genetically used to human immunoglobulin constant regions. The resulting chimeric antibody still contains >30% murine amino acid sequences. Clinical application in humans of chimeric monoclonal antibodies has shown that these proteins retain their immunogenicity in the majority of cases. In another approach, only immunoglobulin variable region sequences relevant for antibody specificity are of murine origin; the constant regions of the immunoglobulin molecule as well as the framework regions of the variable region are of human origin. Clinical application of these "humanized" monoclonal antibodies indicates that these molecules are generally more effective and have no or little intrinsic toxicity or immunogenicity. However, reconstructing the original affinity and specificity in a humanized version of a murine antibody is a time-consuming process and may render the antibody less human. These considerations with chimeric and humanized monoclonal antibodies and the recent clinical success of engineered monoclonal antibodies spurred the development of efficient methods for the isolation and production of fully human monoclonal antibodies, the most desirable antibody format for clinical application.

One method of obtaining human monoclonal antibodies employs transgenic mice harboring human immunoglobulin loci in combination with conventional hybridoma technology. In these mice, large portions of human immunoglobulin heavy and light chain loci have been inserted in the mouse germ line while the endogenous murine immunoglobulin loci have been silenced by gene knockout. Immunization of these transgenic mice with an antigen results in the production of human antibodies specific for the antigen. Human monoclonal antibody-producing cell lines can be obtained from these mice by fusing the spleen cells of immunized mice with plasma cell lines in vitro to obtain immortalized monoclonal antibody-secreting hybridomas. Importantly, production of human antibodies in transgenic mice depends on immunization procedures and is governed by constraints of the murine immune response. As a consequence, it is difficult if not impossible to obtain antibodies against the mouse's own antigens (autoantigens), to xenoantigens that have a high degree of homology to murine autoantigens or to antigens that have poor immunogenic properties such as polysaccharides. These notions have spurred the development of molecular approaches that obviate the need for immunization and cell immortalisation to obtain human monoclonal antibodies with desired specificities. These strategies are based on "immortalisation" of the immunoglobulin genes encoding the antibodies rather than the cell lines producing them.

An alternative method of obtaining human monoclonal antibodies with desirable binding properties employs phage display libraries, an *in vitro,* recombinant DNA-based approach that mimics key features of the humoral immune response. For the construction of phage display libraries, collections of human antibody heavy and light chain variable region genes are expressed on the surface of bacteriophage particles, either in single chain Fv (scFv) or in Fab format. Large libraries of antibody fragment-expressing phages typically contain > 10⁹ antibody specificities and may be assembled from the immunoglobulin V regions expressed in the B lymphocytes of immunized or non-immunized individuals. Alternatively, phage display libraries may be constructed from immunoglobulin variable regions that have been partially assembled in vitro to introduce additional antibody diversity in the library ("semi-synthetic" libraries). For example, *in vitro* assembled variable regions contain stretches of synthetically-produced, randomized or partially-randomized DNA in those regions of the molecules that are important for antibody specificity. Thus, specificities not present in natural repertoires may be added to phage display libraries of antibody fragments.

Recombinant phages expressing antibody fragments of desirable specificities may be selected from a library by one of several methods. Target antigens are immobilized on a solid phase and subsequently exposed to a phage library to allow binding of phages expressing antibody fragments specific for the solid phase-bound antigen. Non-bound phages are removed by washing and bound phages eluted from the solid phase for infection of *Escherichia Coli* (*E.coli*) bacteria and subsequent propagation. Multiple rounds of selection and propagation are usually required to sufficiently enrich for phages binding specifically to the target antigen. Phages may also be selected for binding to complex antigens such as complex mixtures of proteins or whole cells. Selection of antibodies on whole cells has the advantage that target antigens are presented in their native configuration, unperturbed by conformational changes that are introduced by immobilizing an antigen to a solid phase. The constraints imposed by the natural immune response and the influence of the immunogenicity of the target antigen does not permit the isolation of monoclonal antibodies against any antigen by conventional hybridoma technology. In phage approaches, these factors do not play a role, allowing the isolation of monoclonal antibodies against "difficult" antigens such as autoantigens, carbohydrates and toxic antigens.

In one particular selection procedure, phage display libraries are used in combination with flow cytometry and cell sorting to isolate antibody fragments against molecules expressed on the plasma membrane of subpopulations of eukaryotic cells present in a heterogeneous mixture. Thus, the heterogeneous mixture of cells is incubated with the phage library allowing phages to bind to the different cell types. Subsequently, the cells are stained with fluorochrome-labeled monoclonal antibodies to permit identification of the subpopulation of target cells by immunofluorescence analysis and flow cytometry. Target cells and attached phages are collected by flow cytometry and the attached phages are eluted and propagated. This method is rapid, independent of the immunogenicity of the target antigen and yields antibody fragments against molecules in their native configuration. Specific antibodies against very small populations of cells in a heterogeneous mixture can be obtained.

For production of intact human monoclonal antibodies, scFv with desirable specificities can be inserted into mammalian expression vectors containing the genes encoding human immunoglobulin constant regions. We have recently developed a series of constructs that permit the rapid conversion of phage display library-derived scFv antibody fragments to fully human monoclonal antibodys of each immunoglobulin isotype and subclass. Transfected cell lines harboring these constructs produce human monoclonal antibodies *in vitro* that are correctly assembled and glycosylated.

Treatment with monoclonal antibodies or antibody-derivatives directed against tumor surface antigens has proved to be a rational strategy in tumor immunotherapy. Immunotherapy in cancer is aimed at recruiting the humoral and/or cellular arms of the immune system to eradicate tumor cells. To that end, a variety of approaches has been tested in *in vivo* and *in vitro* systems including unconjugated antibodies, bi-specific antibodies, immunotoxins, radiolabeled monoclonal antibodies, immunoliposomes and cytotoxic T lymphocytes. For example, treatment of a large cohort of patients with resected Dukes C colorectal cancer with a murine antibody against the Ep-CAM molecule expressed on colorectal tumor cells has proved very effective. After 7 years of follow-up evaluation, overall mortality of patients was reduced by 32% and the recurrence rate was decreased by 23%. In another example, a humanized antibody against the HER2/neu receptor that is over-expressed on the tumor cells of 25-30% of patients with breast cancer, was shown to slow the progression of tumor growth and to increase the percentage of patients who experienced tumor shrinkage. In another example, a chimeric monoclonal antibody against the CD20 antigen was shown to give a response in 48% of patients with relapsed low grade or follicular lymphoma.

In the above-mentioned examples, treatment of patients with the monoclonal antibody is sufficient for clinical effect. Binding of the antibody to the target antigen results in the recruitment of components of the complement system and effector cells of the immune system with Fc receptor for antibody constant regions that act in concert to kill the tumor cell. For reasons unknown, binding of an antibody to a target antigen on the membrane of a cell does not always result in tumor cell killing. For some antigens it is necessary to arm the monoclonal antibody with entities that kill the tumor cells via other mechanisms such as toxins or radioactive molecules.

In these approaches the specificity of the antibody employed is crucial; ideally, the antibody should specifically bind to tumor cells with minimal cross-reactivity with normal tissues. This criterium is not always met as illustrated by the chimeric anti-CD20 monoclonal antibody. The CD20 antigen is expressed by B cell tumors but also by non-malignant immature and mature B lymphocytes. Despite this cross-reactivity with non-malignant B cells, treatment with the chimeric anti-CD20 antibody causes few side-effects and the clinical success is considerable.

The extracellular matrix controls cell survival, cell morphology and tissue organization by supporting cell adhesion. Remodeling of the extracellular matrix is a key process in the development of properly organised blood vessels, tissues and organs. Many extracellular matrix proteins, such as fibronectin and members of the collagen family, and proteases, such as plasmin and metalloproteinases, are involved in the remodeling process. At the cellular level, remodeling is regulated by members of the "integrin" family of adhesion receptors, which use the extracellular matrix proteins as attachment sites for migration.

A large number of studies have indicated that matrix remodeling and cell migration also play a key role during pathological processes (Werb 1997; Liotta et al. 1991). For example, tumor cell invasion into tissues is dependent on matrix proteolysis. Inhibition of proteases, such as urokinase-type plasminogen activator ("uPA") or metalloproteinases, prevent the formation of metastases (Jankun et al. 1997; Min et al. 1996; Ignar et al. 1998). Matrix remodeling and integrin dependent migration are also crucial for angiogenesis, the formation of new blood vessels (Friedlander et al. 1995). The integrins αvβ3 and αvβ5, in particular, play an important role during angiogenesis (Brooks et al. 1994a). Their expression is elevated on angiogenic endothelial cells and antagonists of αvβ3 suppress tumor growth and can induce tumor regression.

Vitronectin is an important extracellular matrix molecule implicated in the control of cell morphology, proliferation, migration and survival. Because vitronectin is the most important adhesive protein in serum, which is used to culture cells *in vitro,* vitronectin is also called "serum spreading factor". Vitronectin is the primary ligand for αvβ3 and αvβ5 integrins, and plays a prominent role in angiogenesis and tumor growth (Preissner et al. 1997 and 1998). Vitronectin is a 75 kDa glycoprotein that is synthesized primarily in the liver, and is present in plasma in a concentration between 200-400 mg/L (for review see Preissner et al. 1998; Seiffert 1997; Schvartz et al. 1999). In plasma, vitronectin is present in a "native" conformation, whereas in serum, vitronectin is present in its "activated" conformation. Activation of vitronectin can be mediated by a complex of thrombin-antithrombin III and occurs during pathological processes, including angiogenesis. When hyperpermeability is induced by angiogenic factors such as vascular endothelial growth factor ("VEGF"), vitronectin is deposited into the extracellular matrix and forms part of a provisional matrix.

Besides its adhesive function, activated vitronectin binds, concentrates, and regulates proteins that control the formation of the serine protease plasmin. These proteins include plasminogen, plasminogen activator inhibitor type 1 (PAI-1) and the urokinase-type plasminogen activator receptor (uPAR, Kost et al. 1992). By providing a link between plasmin regulated matrix proteolysis and integrin-mediated migration, activated vitronectin appears to play a central role in extracellular matrix remodeling and diseases.

Although several immunohistochemical studies have demonstrated the presence of vitronectin in both normal tissues and in tissues of different pathological origin, the role of activated vitronectin to date has been unclear (Sumiyoshi et al. 1991; Carreiras et al. 1996; Hayman et al. 1983; Loridon-Rosa et al. 1988; Reilly and Nash 1988; Rosenblum and Carsons 1996; Sobel et al. 1995; Sumida et al. 1990; Weller et al. 1991). Studies on the role of activated vitronectin have thus far been hampered by the lack of antibodies that can discriminate between native and activated vitronectin which could, accordingly, be used in immunohistochemical analysis.

In the present invention specific (single chain) antibody fragments, and whole antibodies comprising said fragments, were generated against activated vitronectin using phage display technology. These (scFv) antibody fragments and antibodies were used to determine the role of activated vitronectin in several pathological processes, including tumor growth. Using these fragments and antibodies, the presence and localization of activated vitronectin in various pathological conditions was analyzed. Whereas, in normal tissues, activated vitronectin could hardly be detected, activated vitronectin was abundantly found in the extracellular matrix of various tumors, and in sclerotic kidney as well as in acutely inflamed lung tissue. Activated vitronectin was primarily localized around blood vessels, in large fibrous tissue and in plaques. These results indicate that the activated form of vitronectin is almost exclusively present in disease sites, in particular disease sites where angiogenesis is involved.

Accordingly, the invention includes the specific (single chain) antibody fragments themselves, the corresponding antibodies containing these fragments, and means and methods for making the antibodies and utilizing the antibodies and fragments in the diagnosis and treatment of disease. Diseases to be treated include all diseases involving deposition or presence of activated vitronectin, such as psoriasis, rheumatoid arthritis, artherosclerosis and malignancies. The invention further includes a kit of parts that includes the antibodies or their active fragments.

In one aspect, the invention includes a method of tumor therapy in a subject. The method involves administering to the subject anti-activated vitronectin antibodies and/or antibody fragments which interfere with the adhesion and/or migration of tumor-endothelial cells by preventing vitronectin binding to its receptor, thus providing an antibody-mediated, anti-vascular cancer therapy.

Vitronectin is an important adhesive molecule and, in its activated state as a component of the extracellular matrix, a potential target for tumor therapy. Previous immunohistochemical studies have demonstrated the presence of substantial deposits of vitronectin in most tumors examined and in a number of inflammatory disease states (Carreiras et al. 1996; Loridon-Rosa et al. 1988; Dahlback et al. 1988; Tomasini-Johansson et al. 1998; Guettier et al. 1989; Gladson and Cheresh 1991; Jaskiewicz et al. 1993; Jang et al. 1998). Importantly, however, these earlier studies were performed with antibodies which could not discriminate between native and activated vitronectin. As established herein, to assess the role of vitronectin in angiogenesis, it is important to distinguish between the activated form and the circulating, native form of vitronectin.

By performing subtractive phage selections on urea-activated vitronectin in the presence of plasma as source of native vitronectin, we obtained scFv antibody fragments that discriminated between the two forms of vitronectin. Thus, phage display proved to be a powerful tool to generate scFv antibody fragments that discriminate between the activated and inactivated form of a protein.

We extend the finding that vitronectin deposits are found in most tumors examined and in a number of inflammatory diseases by demonstrating that these deposits are indeed formed by activated vitronectin. The importance of activated vitronectin as a provisional matrix was supported by the virtual absence of activated vitronectin in normal tissue. Of note, activated vitronectin was not exclusively localised in the surroundings of blood vessels, but was present in extra-cellular deposits throughout areas in the tumors and in similar regions in focal glomerular sclerosis and lung tissue from a patient with pneumonia.

Although the precise role of vitronectin in these areas has not yet been fully elucidated, vitronectin is thought to play an important role in the adhesion of (tumor)-endothelial cells to a provisional matrix (Carreiras et al. 1999a) and concentrating proteolytic activity on the cell surface and extracellular matrix by trapping key components, like uPA, uPAR, PAI-1 and plasminogen (Waltz et al. 1997). Furthermore, vitronectin decreases microvascular endothelial cell and tumor cell apoptosis (Isik et al. 1998; Uhm et al. 1999). Thus, activated vitronectin appears to be prominently localized at sites where angiogenesis and tumor cell migration is thought to play a crucial role in tumor growth. Moreover, the fact that vitronectin is normally mainly produced by the liver and that several tumor cell lines also produce vitronectin further indicates that vitronectin plays a role in these conditions (Carreiras et al. 1999b; Tomasini-Johansson et al. 1994; Yasumitsu et al. 1993). Finally platelets, which also contain activated vitronectin (Seiffert and Schleef 1996) and are believed to play an important role in angiogenesis and tumor growth, further stresses an important role for activated vitronectin in pathological processes (Pinedo et al. 1998). Activated vitronectin is the ligand for the αvβ3 integrin, which is upregulated during the angiogenic process and plays an important role in adhesive interactions during angiogenesis (Brooks et al. 1994b). Inhibiting monoclonal antibodies against molecules involved in the adhesion and migration of tumor-endothelial cells have showed promising results in treating tumor-bearing mice (Brooks et al. 1995; Kobayashi et al. 1994; Tsuchiya et al. 1995). Because activated vitronectin binds, concentrates and regulates proteases like plasminogen, PAI-1 and uPAR (Kost et al. 1992), it may be predicted that antibodies specific for activated vitronectin prevent the binding of these proteases thereby inhibiting proteolysis and the invasive potential of endothelial cells. Extrapolating from these results, it may be anticipated that by preventing vitronectin from binding to its receptor a similar result can be obtained. This renders the activated form of vitronectin a potential target for antibody-mediated anti-vascular cancer therapy.

In one aspect the invention provides an antibody fragment comprising at least a CDR3 domain of a heavy chain of an antibody which has a higher affinity for activated vitronectin than for native vitronectin.

Preferably, said antibody fragment is a human or human-like antibody fragment. This renders said fragment more effective and lowers the toxicity or immunogenicity. It is indicated that human or human-like antibody fragments have no or little immunogenicity at all.

More preferably, said antibody fragment is obtainable by subtractive phage selections on urea-activated vitronectin in the presence of plasma as a source of native vitronectin. In one embodiment an antibody fragment of the invention is provided, having a V-region comprising an amino acid sequence of SEQ. I.D. NO. 1, SEQ. I.D. NO. 2, SEQ. I.D. NO. 3, SEQ. I.D. NO. 4 or a functional part, derivative and/or analogue thereof. Of course, one or more amino acid substitutions can be made in the above mentioned sequences, while retaining at least one property of said sequences in kind, not necessarily in amount. It is known to a person skilled in the art, that a substitution of one amino acid with another amino acid with generally similar properties (like size and hydrophobicity) in a certain amino acid sequence does not alter much of the overall functioning of said amino acid sequence. This is called conservative substitution. For instance, a glycine residue may be substituted with an alanine residue. Thus, the invention also provides an antibody fragment, comprising at least 60 percent, more preferably 75 percent, more preferably 85 percent, most preferably 95 percent homology to an amino acid sequence of SEQ. I.D. NO. 1, SEQ. I.D. NO. 2, SEQ. I.D. NO. 3 , SEQ. I.D. NO. 4 or a functional part, derivative and/or analogue thereof.

An antibody fragment of the invention is preferably directed against human activated vitronectin. However, said antibody fragment is also capable of binding to activated vitronectin of other species, as for instance chicken activated vitronectin (example 9).

By an antibody fragment is meant herein a fragment of at least the size of a CDR3 domain, in particular a fragment derived from a CDR3 of a heavy chain. This fragment has at least one same property in kind, not necessarily in amount, as said antibody.

A higher affinity of a fragment for activated vitronectin than for native vitronectin means herein that said fragment binds more activated vitronectin than native vitronectin when said fragment is exposed to the same excess amount of both kinds of vitronectin. This can be measured in a mixture, comprising equal excess amounts of activated and native vitronectin. This mixture can be provided to a certain amount of the same kind of antibody fragments. After incubation it is possible to determine the amount of bound activated vitronectin and bound native vitronectin. If it is then concluded that said antibody fragments have bound more activated vitronectin than native vitronectin, said antibody fragments are considered to have a higher affinity for activated vitronectin than for native vitronectin.

A functional part of a protein, for instance a functional part of an antibody, is defined herein as a part which has the same kind of properties in kind, not necessarily in amount. Said properties may for instance comprise the capability to bind to a specific antigen. A functional derivative of a protein is defined as a protein which has been altered such that the properties of said molecule are essentially the same in kind, not necessarily in amount. A derivative can be provided in many ways, for instance through conservative amino acid substitution. For instance, one or more glycine residues may be substituted for alanine, which will not alter much of the properties of said protein.

A person skilled in the art is well able to generate analogous compounds of a protein, for instance an antibody. This can for instance be done through screening of a peptide library. Such an analogue has essentially the same properties, for instance binding properties, of said protein in kind, not necessarily in amount.

Further provided is an antibody comprising an antibody fragment of the invention. Preferably, said antibody is suitable for being provided to the blood circulation of an individual. In that case, a target molecule for said antibody can be reached via the blood circulation. Of course, said antibody must be sufficiently stable to remain in the blood circulation until said target molecule is reached. As is known to a person skilled in the art, IgG antibodies are very suitable for providing to the blood circulation of a subject. Thus, in a preferred aspect the invention provides an antibody of the invention which is an IgG antibody. The construction and production of an IgG₁ antibody comprising an antibody fragment of the invention is described in example 4. An antibody or fragment of the invention can, for instance, be used to diagnose a disease state in a subject, for instance through detecting the presence of activated vitronectin in a biological sample taken from said subject.

In another aspect the invention provides a pharmaceutical composition comprising an antibody fragment according to the invention. Such composition can be used in an anti-vascular therapy, preferably, a cancer therapy. The invention therefore also provides the use of an activated form of vitronectin as a target for antibody-mediated anti-vascular cancer therapy. In one embodiment a method of cancer therapy is provided, comprising administering anti-activated vitronectin antibody fragments or antibodies according to the invention, which interfere with the adhesion and/or migration of tumor-endothelial cells, to a patient.

Now that a role of activated vitronectin in the development of certain diseases, for instance cancer, is established, activated vitronectin can be used as a marker to detect said diseases. Thus, in one aspect the invention provides a method of detecting the presence of activated vitronectin comprising using an antibody fragment or an antibody of the invention. The use of an antibody fragment or an antibody of the invention for the detection of the presence of activated vitronectin is also herewith provided.

In another aspect the invention provides a method of diagnosing a disease state in a subject, said method comprising detecting for the presence of activated vitronectin in a biological sample taken from said subject. Preferably, said activated vitronectin is detected with use of an antibody and/or an antibody fragment, more preferably with use of an antibody and/or an antibody fragment of the invention. In one embodiment said detection of activated vitronectin comprises detecting the presence of said antibody, antibody fragment, or a part thereof, bound to said vitronectin. A lot of techniques are known in the art for detecting the presence of an antibody, for instance bound to activated vitronectin. Said antibody may for instance be labeled with a fluorescent or radioactive label. After said antibody has been allowed to bind to activated vitronectin, unbound antibody can be removed by one or more washing steps. After that, a fluorescent or radioactive signal will reveal the presence of bound antibody and, hence, the presence of activated vitronectin. Of course, a person skilled in the art can think of many alternative ways to detect the presence of an antibody, for instance bound to activated vitronectin.

Now that it is shown to be possible to detect an activated form of vitronectin, a person skilled in the art is put in the position that he/she is able to devise strategies for finding additional binding molecules specific for activated vitronectin using means and methods available in the art. The invention therefore also provides a method of manufacturing an antibody or an antibody fragment directed toward detecting the presence of activated vitronectin. Such method can include the use of an apparatus for detecting the presence of activated vitronectin, derived by selection from a library using a phage display method. The invention therefore also provides such an apparatus. In another embodiment the invention provides an apparatus for detecting the presence of activated vitronectin, using an antibody or an antibody fragment, said antibody or fragment derived by selection from a library comprising urea-treated vitronectin capable of binding to an antibody or antibody fragment of interest, such binding forming a receptor complex and thereby causing a change in a property associated with the binding partner; and a detection means for detecting the measurable change. Preferably, the receptor complex comprises an antibody or an antibody-binding fragment thereof, a peptide or a nucleotide sequence. In another preferred embodiment the receptor complex comprises an antibody or an antibody-binding fragment thereof derived by selection from a library using a phage display method.

The invention further provides an isolated peptide fragment comprising at least a CDR3 domain of a heavy chain of an antibody which has a higher affinity for activated vitronectin than for native vitronectin. Preferably, said peptide fragment is obtainable by subtractive phage selections on urea-activated vitronectin in the presence of plasma as a source of native vitronectin. Mutants of a peptide of the invention can be easily tested for the same binding characteristics in kind, not necessarily in amount, and are therefor also part of the invention.

Activated vitronectin is the most important adhesive protein in serum. Binding of an antibody fragment of the invention to said vitronectin will therefore affect the capability of said vitronectin to bind to a lot of different targets. Said target may for instance be an endothelial cell, preferably a tumor endothelial cell, but may also for instance be platelets. Thus, in one aspect the invention provides a use of an antibody or an antibody fragment according to the invention for at least in part changing an adhesion property of activated vitronectin.

Said adhesion property preferably comprises the capability to bind to endothelial cells, extracellular matrix proteins, and/or platelets. In a more preferred embodiment, said changing of the capability to bind to extracellular matrix proteins prevents angiogenesis.

Now it is possible, using an antibody fragment of the invention, to at least in part change the capability of activated vitronectin to bind to endothelial cells and/or collagen. Thus one embodiment of the invention provides a method for at least in part inhibiting adhesion of an endothelial cell to activated vitronectin, comprising providing said vitronectin with an antibody fragment, an antibody or a peptide according to the invention.

Another embodiment of the invention provides a method for at least in part decreasing an interaction of activated vitronectin with collagen and/or endothelial cells, comprising providing said vitronectin with an antibody fragment, an antibody or a peptide according to the invention.

Another property of activated vitronectin is it's capability to decrease microvascular endothelial cell and tumor cell apoptosis (Isik et al. 1998; Uhm et al. 1999). This can lead to unlimited cell growth and division, which leads for instance to tumor development and/or angiogenesis. This highly undesirable effect of activated vitronectin to decrease cell death can now be counteracted by an antibody fragment, an antibody and/or a peptide of the invention. Thus, in one aspect the invention provides a method for at least in part inducing cell death of an endothelial cell, associated with an extracellular matrix comprising activated vitronectin, comprising providing said matrix with an antibody fragment, an antibody or a peptide according to the invention. Said cell death may comprise apoptosis.

In yet another aspect the invention provides an isolated peptide comprising a vitronectin epitope which is recognized by an antibody fragment, an antibody or a peptide according to the invention. Said peptide may for instance be useful for screening a library for their binding properties to said epitope and, hence, for activated vitronectin. As is described in example 8, the vitronectin H2 domain is an important epitope for an antibody fragment of the invention. Thus, in a preferred aspect the invention provides a peptide according to the invention, which comprises at least part of the vitronectin H2 domain.

Now that an antibody fragment of the invention is provided, a person skilled in the art is well capable of generating a nucleic acid molecule encoding said antibody fragment. Methods to generate nucleic acid molecules encoding a certain polypeptide, starting from the amino acid sequence of said polypeptide, are known in the art, as well as codon usage in different organisms. Hence this needs no further explanation here. Thus the invention provides an isolated or recombinant nucleic acid molecule encoding an antibody fragment, an antibody or a peptide according to the invention, or a functional part, derivative and/or analogue thereof.

Of course, a person skilled in the art is equally well capable of generating a nucleic acid molecule encoding a peptide comprising a vitronectin epitope which is recognized by an antibody fragment, an antibody or a peptide according to the invention, or a functional part, derivative and/or analogue thereof. Said nucleic acid molecule is also herewith provided.

Preferably, said epitope comprises at least part of the vitronectin H2 domain. With methods known in the art the artisan is also capable of generating a fusion gene comprising a nucleic acid molecule of the invention. Said fusion gene may encode a fusion protein. Thus, in one aspect the invention provides a fusion protein comprising a peptide encoded by a nucleic acid molecule of the invention.

By at least a functional part of a nucleic acid of the invention is meant a part of said nucleic acid, at least 30 base pairs long, preferably at least 200 base pairs long, comprising at least one expression characteristic (in kind not necessarily in amount) as a nucleic acid of the invention.

During the process of angiogenesis and tumor growth, platelets, which contain activated vitronectin, are also believed to play an important role. Therefore said platelets are also an important target for antibody-mediated anti-vascular cancer therapy. As is described in example 10, an antibody fragment of the invention is able to reduce the activation of platelets. Thus one embodiment of the invention provides a method for at least in part inhibiting platelet aggregation in the presence of vitronectin, comprising providing said vitronectin with an antibody fragment, an antibody or a peptide of the invention.

With the teachings of the present invention it is clear that an antibody fragment, an antibody or a peptide of the invention is very suitable for the preparation of a medicament. A use of an antibody fragment, an antibody or a peptide of the invention for the preparation of a medicament is therefore also provided. Of course, said medicament is particularly suited for at least one of the above mentioned applications. Thus, in one preferred aspect, a use of an antibody fragment, an antibody or a peptide of the invention for the preparation of a medicament for at least in part inhibiting platelet aggregation is provided. In another preferred aspect, a use of an antibody fragment, an antibody or a peptide of the invention for the preparation of a medicament for at least in part decreasing an interaction of activated vitronectin with collagen and/or endothelial cells is provided. Another preferred embodiment provides a use of an antibody fragment, an antibody or a peptide of the invention for the preparation of a medicament for at least in part changing the development of blood vessels.

Activated vitronectin is an important extracellular matrix protein implicated in the control of cell morphology, proliferation, migration and survival. Activated vitronectin is for instance involved in the development of blood vessels. With the teaching of the present invention it is possible to change said development of blood vessels. This can be done by influencing the remodeling process of the extracellular matrix of an endothelial cell. Thus, one embodiment provides a method for at least in part changing the development of blood vessels, comprising influencing the remodeling process of the extracellular matrix of an endothelial cell. In a preferred embodiment, said extracellular matrix is provided with an antibody fragment, an antibody or a peptide according to the invention.

Now that an antibody fragment with a higher affinity for activated vitronectin than for native vitronectin is provided, the artisan is well capable of designing an antibody fragment, preferably based on a fragment of the invention, with a same binding property in kind, not necessarily in amount. Such fragment is capable of at least in part inhibiting binding of an antibody fragment, an antibody or a peptide of the invention, to activated vitronectin. By at least in part inhibiting binding of a molecule to activated vitronectin is meant herein that less molecules will bind to a certain limiting amount of activated vitronectin if an inhibitor is also provided. If a limiting amount of activated vitronectin is provided with a certain amount of said molecules and an inhibitor, at least a part of said inhibitor will bind to said activated vitronectin. The inhibitor-bound activated vitronectin is not capable of binding to said molecules anymore. This results in less amount of said molecules to be bound to vitronectin, as compared to a situation wherein no inhibitor was provided.

Thus, in one aspect, the present invention provides an antibody or functional part, derivative and/or analogue thereof, capable of at least in part inhibiting binding of an antibody fragment, an antibody or a peptide of the invention, to activated vitronectin.

### MATERIALS AND METHODS

A phage antibody library cloned in the phagemid pPVT with a size of 4.5 x 10⁹ specificities was used. Urea treated vitronectin was prepared as described earlier (Yatohgo et al. 1988). Native vitronectin was a gift from Dr K.T. Preissner (Bad Nauheim, Germany). The rabbit polyclonal antibody and the monoclonal antibody 13H1 against vitronectin have been previously described (Preissner et al. 1987). The monoclonal antibody anti-[VSV-G]-peroxidase and anti- [c-myc] -peroxidase were obtained from Roche and anti-CD31 monoclonal antibody EN4 from Monosan. The peroxidase-conjugated rabbit anti-mouse polyclonal antibody was obtained from Dako.

### Phage selection procedures

Phage selections were performed as described with minor modifications (De Kruif et al. 1995). Briefly, immunotubes (Gibco BRL) were coated with urea treated, activated vitronectin (10 µg/ml) overnight at 4°C and blocked with 2% milk powder in PBS (MPBS) for 2 hours. Before the phage were added to the immunotubes, they were blocked for 15 minutes with MPBS containing 50% human pooled plasma to absorb non-specific binding phages and phages recognizing native vitronectin. The immunotubes were incubated with phages for 2 hours at room temperature. After extensive washes with PBS containing 0.1% Tween-20 followed by washings with PBS alone, bound phages were eluted with 1 ml 0.1M HCl for 5 minutes at room temperature. The mixture was neutralized with 500 µl 1 M Tris-HCl pH 7.4. Eluted phage were used to infect *E.coli* XL1-blue (Stratagene) and the bacteria were plated on TYE medium containing the appropriate antibiotics and glucose. After overnight culture at 37°C, bacterial colonies were scraped and the bacteria were used to prepare the next library.

After three rounds of selections, monoclonal phages were prepared from single ampicillin and tetracycline-resistant colonies of infected XL1-Blue cells. Binding to urea treated vitronectin was tested in an ELISA using a phage antibody detection module (Pharmacia, Inc.). A monoclonal anti-M13 antibody (Pharmacia, Inc.) was used and the assay was performed according to manufacturer instructions. Nucleotide sequence analysis was determined with the ABI PRISM Dye Terminator Cycle sequencing kit (Perkin Elmer) on an automated sequencer.

### Preparation and purification of scFv fragments

The plasmids of the pPVT phagemids were digested with NotI and NcoI restriction enzymes and the coding regions for the scFv's were subcloned in the NotI/NcoI digested vector IM3-HIS that adds 6 histidine residues (HIS-tag) and a myc tag to the carboxyterminus of the scFv fragment (De Kruif et al. 1996). scFv fragments were produced in the *E.coli* non-suppressor strain SF110. Periplasmic preparations of scFv's were purified over a Ni-NTA agarose column (Qiagen) and eluted by imidazole into 2 ml fractions. The purified scFv antibody fragments were dialysed against PBS and stored at - 20°C until further use.

### ELISA's

scFv antibody fragments (30 µg/ml) or a control polyclonal anti-vitronectin antibody (5µg/ml) were coated overnight on 96 well plates (Nunc Maxisorp). Purified activated or native vitronectin was allowed to bind for 1 hour at room temperature. After washing with PBS/0.1% Tween-20, bound vitronectin was determined by incubation with murine monoclonal antibody 13H1 which recognizes both native and activated vitronectin (Kost et al. 1992). After washing and incubation with a peroxidase-conjugated rabbit anti-mouse polyclonal antibody, staining was performed with 2,2'-azino-di[3-ethyl-benzthiazoline-sulfate] (Roche) and H₂O₂ in 0.05 M citric acid (pH 4.0). Optical density was read at 405 nm in a microtiter-plate reader (Molecular Devices Corp).

For the competition ELISA, plasma was used as a source of native vitronectin and serum as a source of activated vitronectin. Urea activated vitronectin (5 µg/ml) was coated overnight in a 96 well microtitre plate. Phage or scFv's were pre-incubated with plasma or serum for 30 min. The pre-blocked phage and scFv's were allowed to bind to coated vitronectin for 1 hour at room temperature. After washing with PBS/0.1% Tween-20, binding was determined by incubation with a peroxidase-conjugated monoclonal anti-M13 antibody (phages) or anti-myc (scFv's). Staining was performed and measured as described above. The ELISA's were performed in duplicate or triplicate and repeated at least two times.

### Immunohistochemistry

Immunohistochemical studies were performed on tissues from patients with colon-, kidney- or lung cancer and patients with pneumonia or focal nephro- sclerosis. Also a-term placental tissue was included. Tissue samples were immediately frozen in liquid nitrogen after surgical resection and 6 µm thick cryostat sections were prepared. Analysis was performed after fixation in acetone/0.35% H₂O₂ for 10 minutes. The cryostat sections were air dried for 1 hour and blocked for 1 hour with 4% MPBS/1% BSA. The sections were subsequently incubated with the scFv antibodies or monoclonal antibody EN4 (áCD31) for 1 hour and washed with PBS/0.1% Tween-20, followed by incubation for 1 hour with the peroxidase-conjugated monoclonal antibody anti-VSV-tag or peroxidase-conjugated rabbit anti-mouse polyclonal antibody. Anti-thyroglobulin scFv antibody fragment was used as negative control (De Kruif et al. 1995). The conjugate was developed using diaminobenzidine (Graham and Karnovsky 1966).

### Affinity measurements

The Biacore 2000 system and the reagents, including the sensorchips CM5, surfactant P20 and the amine coupling kit, containing N-hydroxy-succinimide (NHS), N-ethyl-N'-(dimethanylamino-propyl)carbidiimide (EDC), ethanolamine hydrochloride and formeate buffer, were from Pharmacia Biosensor AB (Uppsala, Sweden). The whole procedure was performed according to the manufacturers instructions. Briefly, in separate BiaCore flow cells, approximately 7000, 5000 and 2500 resonance units of purified urea treated vitronectin in 10mM formate buffer (pH 3.0) were coupled to a CM5 sensor chip using NHS /EDC amine coupling chemistry. Association and dissociation were measured under continuous flow of 30 µl/min using a concentration range of the anti-vitronectin huMabs from 10 to 100 nM. The association was determined from a plot of (ln(d0/DT)t) versus concentration.

### Preparation of endothelial cells

Human Umbilical Vein Endothelial Cells (HUVEC) were isolated from umbilical cords using trypsin/EDTA solution, which was applied to the vein using special canules known to persons skilled in the art. The solution was incubated in the vein for 15 min, the detached endothelial cells were flushed out and washed once with RPMI-1640 medium supplemented with a cocktail of general antibiotics and 20% (v/v) of human serum. For the adhesion and apoptosis assay described below, HUVEC's of the second passage were used. Endothelial cells (HUVEC) were brought in suspension with trypsin, trypsin was neutralized in medium containing serum and cells were washed once and resuspended in RPMI-medium containing 1% bovine serum albumin. Cells were counted using a Bürker-Türk chamber and for the adhesion assay 5x10⁴ cells were seeded in 96-well plates in the presence of anti-VN antibodies or a control antibody named L1R3.

### Adhesion assay

To estimate attached cell numbers, a chromogenic substrate for the ubiquitous lysosomal enzyme, hexosaminidase was used. The protocol for the adhesion assay has been described previously (Landegren 1984). Briefly, urea-treated vitronectin was coated to wells of a 96 well plate overnight (5 µg/ml). Plates were blocked with phosphate buffered saline containing 1 mM calciumchloride, 1 mM magnesium-chloride (PBS⁺⁺) and 3% bovine serum albumin (BSA) for 2 hours. HUVEC cells were added to the wells (5x10⁴) in the presence of the antibodies against activated vitronectin or control antibodies and allowed to adhere for 2 hours at 37°C in a 5% CO₂ humidified incubator. The plates were washed four times with PBS⁺⁺/3% BSA. Hundred µl of 7.5 mM of p-nitrophenol-N-acetyl-β-D-glucoseaminide in 0.1 M citrate buffer, pH 5, mixed with an equal volume of 0.5% Triton X-100 in water [NPAG/TX100]) was added to the wells and incubated for 90 minutes at 37°C in a 5% CO₂ humidified incubator. Enzyme activity was stopped by addition of 150 µl 50 mM glycine buffer, pH 10, containing 5 mM EDTA. Absorbance was measured at 405 nm in a microtiter-plate reader (Molecular Devices Corp, Sunnyvale, CA, USA).

### Apoptosis assay

Annexin-V and propidium iodide were used for apoptosis measurement. Urea-treated vitronectin was coated overnight to wells of a 48 well plate (250 µl, 5 µg/ml). HUVEC was added (5.10⁴ cells/well) and allowed to adhere for 30 minutes. Subsequently, the anti-vitronectin antibodies or a control antibody were added with a concentration of 50 µg/ml and incubated at 37 °C in a 5% CO₂ humidified incubator. At different time points the cells were washed and trypsinised. Apoptosis was determined by flow cytometry using the annexin-V-fluos staining kit from Roche.

### Binding assays

PAI-1 was a gift of Dr Pannekoek (Amsterdam, The Netherlands) and uPAR from Dr Meijers (Amsterdam, The Netherlands). A stock solution of 50 µg/ml TAT was formed by incubation of 20 µg/ml with 5 times molar excess of ATIII. No binding was observed of VN to ATIII alone, so the excess of ATIII was not removed. Human Collagen type I (Sigma) was dissolved in 0.5 M acetic acid (1 mg/ml) and further diluted in PBS. Plasminogen was affinity purified from outdated plasma using lysine-Sepharose (Pharmacia). The vitronectin ligands PAI-1, plasminogen, uPAR, TAT and collagen type I were coated overnight to 96-well plates in PBS (5 µg/ml). Phosphatidyl serine (PS, Sigma) was dissolved in methanol at a concentration of 50 µg/ml and 50 µl/well was air-dried. Coated ligands were blocked with PBS containing 3% BSA and 0.1% Tween-20 (in the case of PS, Tween-20 was omitted) and subsequently incubated with urea-treated vitronectin (0-100 µ g/ml). Binding of vitronectin was determined with a polyclonal antibody, followed by peroxidase conjugated swine anti-rabbit IgG and peroxidase substrate (ortho-phenylenediamine). The concentration that yielded half-maximal vitronectin binding was calculated. In the competition experiments, this half maximal concentration of vitronectin was used and pre-incubated (30 minutes at 37°C) with increasing amounts of Vn18 IgG or control IgG (0-100 µg/ml). Binding of vitronectin was determined as described above. The effect of VN18 was expressed as percentage of binding of vitronectin to its ligands in the presence of control IgG. For heparin binding experiments, urea-treated vitronectin (5 µg/ml) was coated overnight, blocked and incubated with increasing amounts of biotinylated-heparin (0-100 µg/ml). Heparin binding was determined with peroxidase-conjugated streptavidin in the presence of peroxidase substrate. Half-maximal heparin concentrations were used and different concentrations of VN18 or control IgG (0-100 µg/ml). Residual heparin binding was expressed as percentage binding of control IgG.

### EXAMPLES

### Example 1. Identification of phage antibodies that specifically recognize activated vitronectin.

To isolate scFv antibodies against the activated vitronectin phage display on immunotubes coated with urea treated human vitronectin was deployed. It has previously been shown that urea treatment leads to multimerisation of vitronectin, which resembles the conformation of activated vitronectin in serum (Yatohgo et al. 1988). To subtract phage that also bind native vitronectin, phage selections were performed in the presence of plasma as a source of native vitronectin. After three rounds of selection, 48 monoclonal phage antibodies (mophabs) were tested for binding to urea-treated vitronectin by ELISA. One third of the phage were scored positive and selected for further analysis. Nucleotide sequence analysis of the V-regions encoded by these phages unveiled the presence of four independent phage antibodies, which we named Vn1, Vn7, Vn18 and Vn26 (Table I).

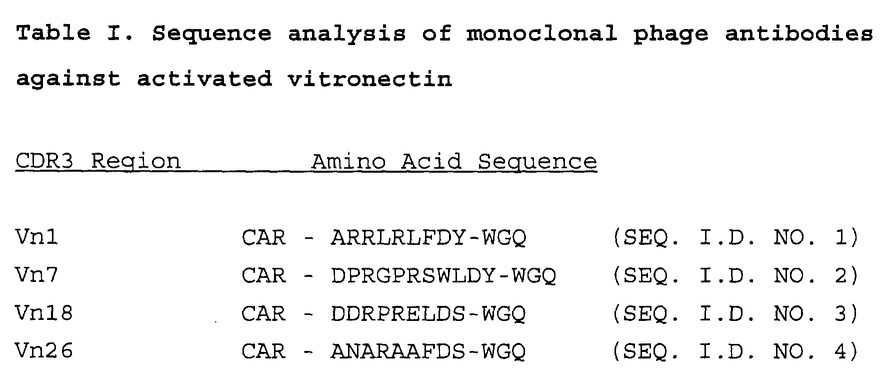

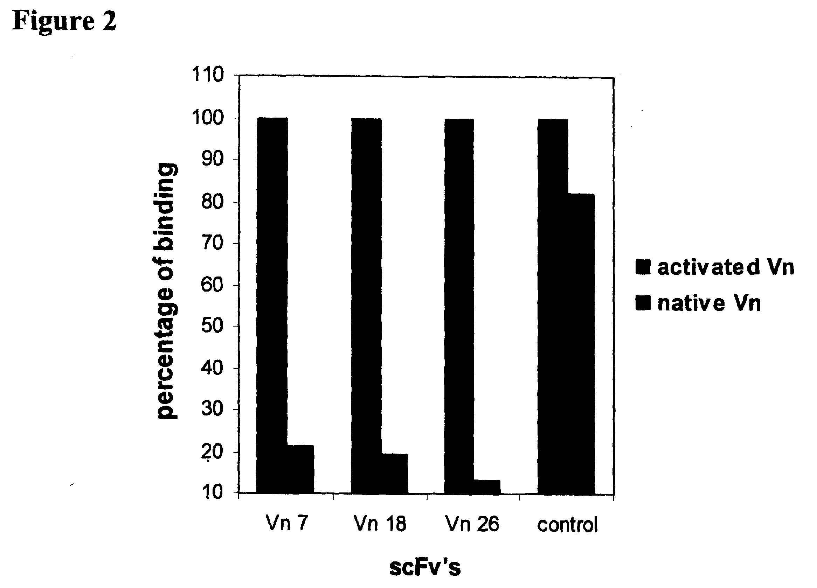

It was investigated whether scFv's derived from the monoclonal phage antibodies specifically recognized activated vitronectin. scFv's dilutions were used that yielded maximum binding. Since native vitronectin becomes activated upon coating to plastic, a capture ELISA was performed. A rabbit polyclonal anti-vitronectin antibody served as a non-specific control and was coated and used to capture native or activated vitronectin. As can be seen in Figure 2, scFv(Vn7), scFv(Vn18) and scFv(Vn26) specifically detected activated vitronectin. Unfortunately, scFv's derived from phage Vn1 could not be analyzed, because the anti-VSV monoclonal antibody did not detect scFv(Vn1). A possible explanation could be the presence of a mutation in the sequence encoding the VSV-tag. As expected, the control polyclonal anti-vitronectin antibody recognized activated and native vitronectin equally well.

### Example 2. Recognition by monoclonal phage antibodies and scFv fragments of activated vitronectin in serum as compared to native vitronectin present in plasma.

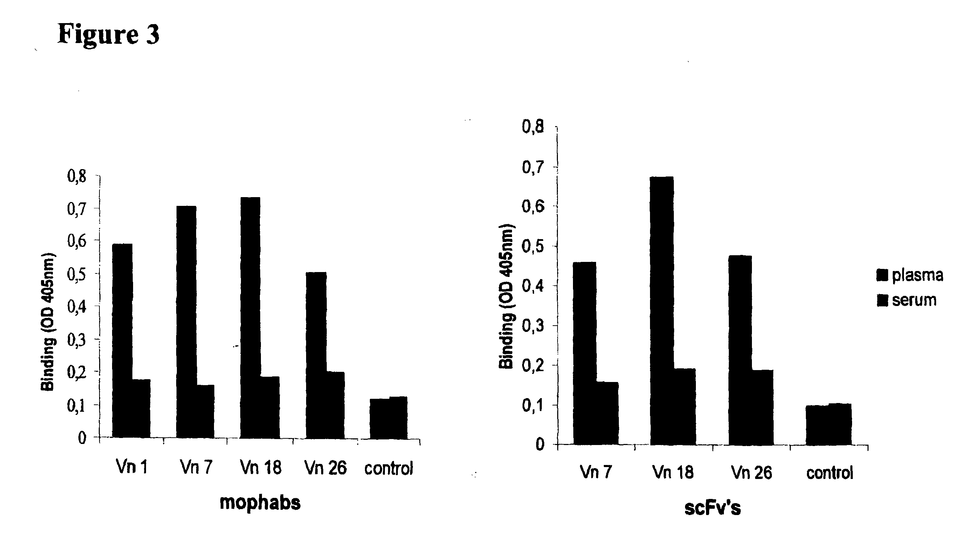

It was investigated whether the anti-vitronectin mophabs and scFv's recognized activated vitronectin present in serum. Phage and scFv's were allowed to bind to coated urea-treated vitronectin in the absence or presence of plasma or serum. As can be seen in Figure 3, binding of all mophabs and scFv's was inhibited by serum, but not by plasma. These results strongly suggest that the phages and scFv's also specifically recognize physiologically activated, serum vitronectin, but not native, plasma vitronectin.

### Example 3. Recognition of vitronectin by Vn18 in normal and tumor tissue sections.

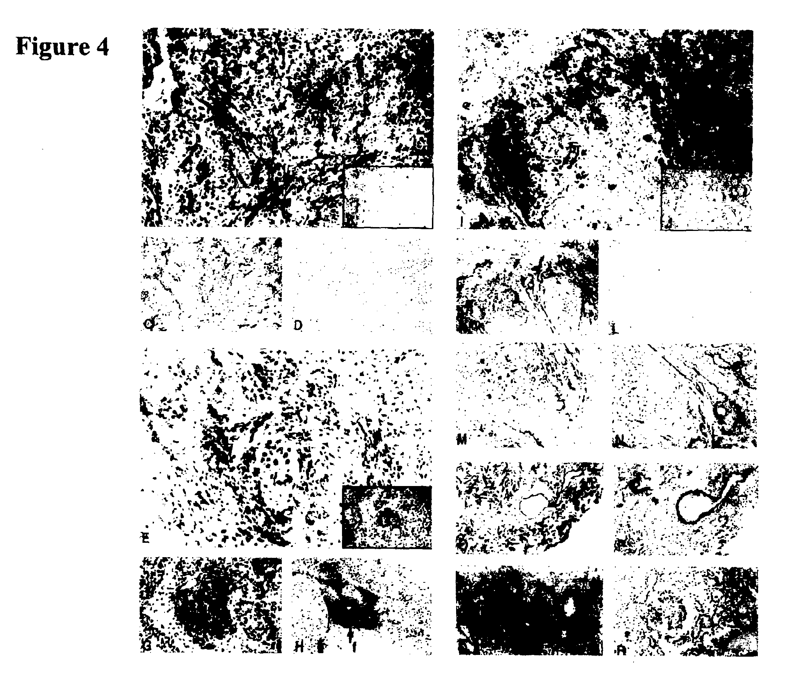

scFv(Vn18) was selected to study the presence and distribution of activated vitronectin in human carcinoma of colon, kidney and lung, in other pathological specimens, and in the corresponding normal tissues. Activated vitronectin was abundantly present in colon adenocarcinoma (Figure 4A), whereas hardly any activated vitronectin could be detected in normal colon (Figure 4D). The staining pattern of scFv(Vn18) overlapped largely with the pattern of staining with antibodies against CD31 (Figure 4B), an endothelial specific marker, indicating the presence of activated vitronectin around blood vessels. In addition to the staining around blood vessels, staining of vitronectin deposits was seen in the extracellular environment, fibrous tissue, where vessels had not yet been formed.

In renal cell carcinoma activated vitronectin was mainly present at the rim of the tumor (Figure 4E). The scFv(Vn18) staining pattern in these regions is similar to "CD31 staining (Figure 4F), indicating the presence of activated vitronectin around blood vessels. In contrast, in the middle of the tumor hardly any activated vitronectin was detected, although "CD31 staining revealed the presence of blood vessels (data not shown). In regions of the kidney which were not affected by the tumor hardly any staining for activated vitronectin was seen (Figure 4H). In contrast, the affected glamorously in kidneys with focal glomerular sclerosis stained strongly positive (Figure 4N), whereas the normal, unaffected glamorously were negative (Figure 40). In non-small cell lung cancer, similar scFv(Vn18) staining patterns were observed (Figure 4I-K). Finally, in an a-term placenta, fibrous plaques were positive for activated vitronectin as well as small capillary-like structures (Figure 4P).

### Example 4. Construction and production of a fully human monoclonal IgG₁/κ antibody against activated vitronectin.

Construction of eukaryotic expression vectors and production of human monoclonal antibodies (huMabs) has been described in detail elsewhere (Huls et al. 1999). Briefly, in a two-step cloning procedure, the V_{H} and V_{L} regions encoding a scFv were cloned into the vector pLEADER to add the T-cell receptor α-chain leader HAVT20 leader peptide sequence and a splice donor site. Next, the V_{H} and V_{L} regions, which contain leader and splice donor sites, were subcloned in the pNUT-Cγ1 or pNUT-Cκ expression vectors. HuMab Vn7, Vn18 and Vn26 were produced by establishing stably transfected BHK21 cells. Cells were maintained at 37°C in a 5% CO₂ humidified incubator in Iscove's modified Dulbecco's medium containing 10% FCS, 2 mM glutamine, 100 U/ml penicillin and 100 µg/ml streptomycin. Recombinant antibodies were purified from approximately 2 liters of culture supernatant by protein A sepharose affinity chromatography and run on an SDS-PAGE gel to confirm the stoichiometry and quality of heavy and light chains as described (Huls et al. 1999). Concentration of purified huMab was determined by spectophotometry at 280 nm.

### Example 5. Inhibition of endothelial cell adhesion to vitronectin.

For all functional studies, scFv's were converted to intact, fully human IgG1 monoclonal antibodies by recloning the V_{H} and the V_{L} regions into expression vectors for synthesis of complete IgG₁/κ molecules in BHK cells. Purified antibodies retained their specificity for activated vitronectin as assessed in ELISA. The kinetic association and dissociation rates and affinity constant of the three huMabs were determined by surface plasmon resonance on a BiaCore. Association and dissociation constants ranged from 1.5x10⁵ - 4.6x10⁵ M⁻¹S⁻¹ and 6.8x10⁻⁴ - 2.9x10⁻² S⁻¹ respectively, yielding affinity constants of 4.5, 9.3 and 92 nM for Vn7, Vn18 and Vn26.

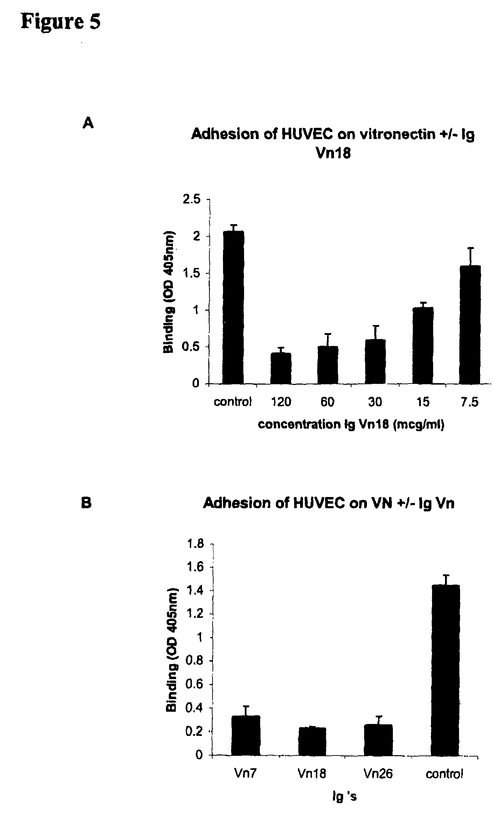

Vitronectin is believed to play a prominent role during angiogenesis (Preissner et al. 1997). To evaluate the consequences of binding of human anti-vitronectin antibodies to activated vitronectin on endothelial function, a number of assays were performed. First, the effect on endothelial cell adhesion was analysed. Culture dishes were coated with activated vitronectin and endothelial cells and serial dilutions of anti-vitronectin antibodies or a control antibody were simultaneously added to each well. Endothelial cells (HUVEC's) were allowed to adhere and after washing, numbers of adhering cells were estimated by adding a chromogenic substrate for an ubiquitous lysosomal enzyme to the wells (Landegren 1984). Adherence of endothelial cells to vitronectin-coated plates was inhibited with anti-vitronectin antibodies in a dose dependent fashion (Figure 5A). All three anti-vitronectin antibodies inhibited endothelial cell adhesion (Figure 5B). Maximum inhibition of endothelial cell binding was 80-85% at concentrations of anti-vitronectin antibodies of 50 µg/ml.

### Example 6. Effect of anti-vitronectin antibodies on endothelial cell apoptosis.

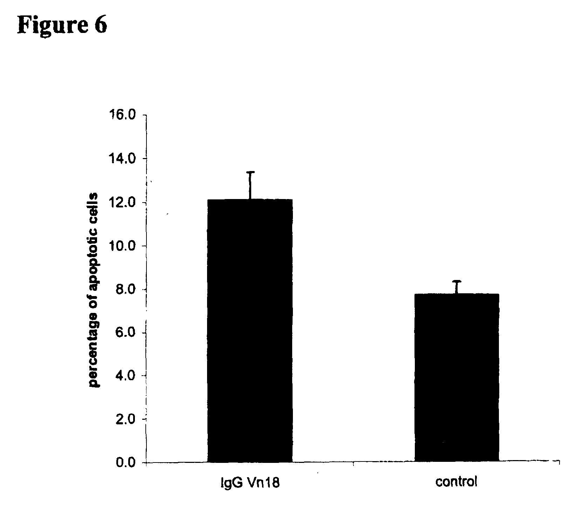

Vitronectin protects endothelial cells from entering an apoptotic pathway (Isik et al. 1998). To evaluate a possible effect of human anti-vitronectin antibody Vn18 in inducing endothelial cell apoptosis, anti-vitronectin antibodies or a control antibody were added to subconfluent layers of endothelial cells (HUVEC's). After 8 hours, cells were stained with propidium iodide and annexin V and analysed by flow cytometry. In Figure 6 it is shown that the number of apoptotic cells increased as a result of binding of the Vn18 antibody to vitronectin-coated plates.

### Example 7. Effect of anti-vitronectin antibodies on vitronectin function, mapping of the epitope and cross-reactivity with vitronectin from other species.

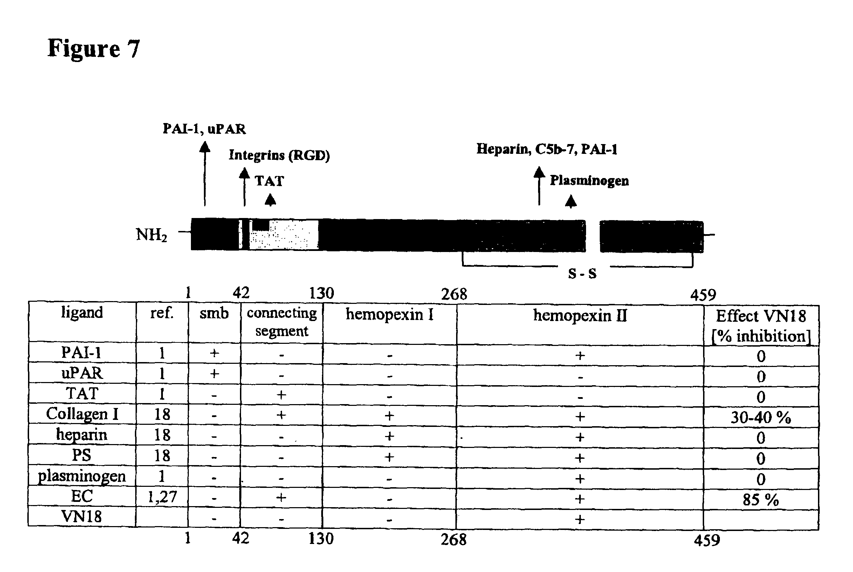

In addition to a role in cell adhesion, vitronectin is involved in the regulation of protease cascades, such as coagulation and fibrinolysis (Preissner et al. 1997). To evaluate a potential effect on these systems the capacity of anti-vitronectin antibodies to inhibit interactions of vitronectin with its ligands was determined (Preissner and Seiffert 1998; Schvartz et al. 1999). Binding assays were performed essentially as described in the materials and methods. The results are summarized in Figure 7. Vn18 reduced binding of vitronectin to collagen and inhibited attachment of endothelial cells almost completely. Binding of six other vitronectin ligands was not affected.

### Example 8. Mapping of the epitope on vitronectin that is recognized by Vn18.

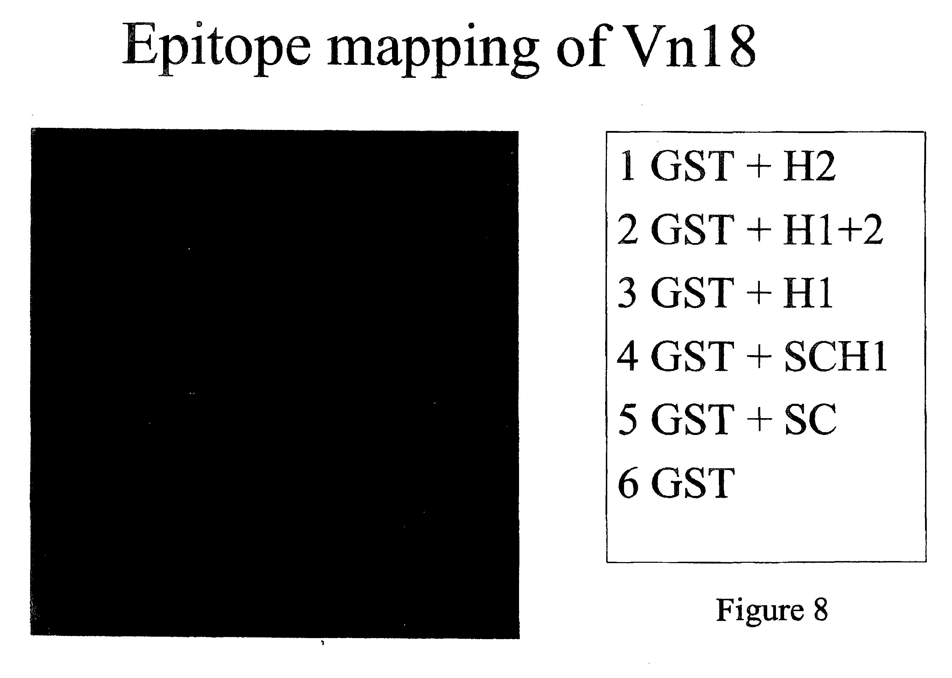

In order to determine the specific epitope for binding of the antibody to vitronectin, vitronectin fragments as described by Yoneda et al. (1998) were used. Immunoblot analysis of recombinant vitronectin fragments revealed binding of Vn18 antibodies to an epitope located in the hemopexin II domain (Figure 8). For this, several GST-fusion proteins were purified and let to interact with Vn18 antibodies. Domains that are present in vitronectin are S (aa. 1-42), C (aa. 43-130), H1 (aa. 131-286) and H2 (aa. 287-459). The subdivision into these four domains is depicted in Figure 7. The result shown in Figure 8 suggests that Vn18 specifically recognizes a domain near the N-terminus of vitronectin: the H2 domain (aa. 287-459).

### Example 9. Cross-reactivity of Vn18 with vitronectin from other species.

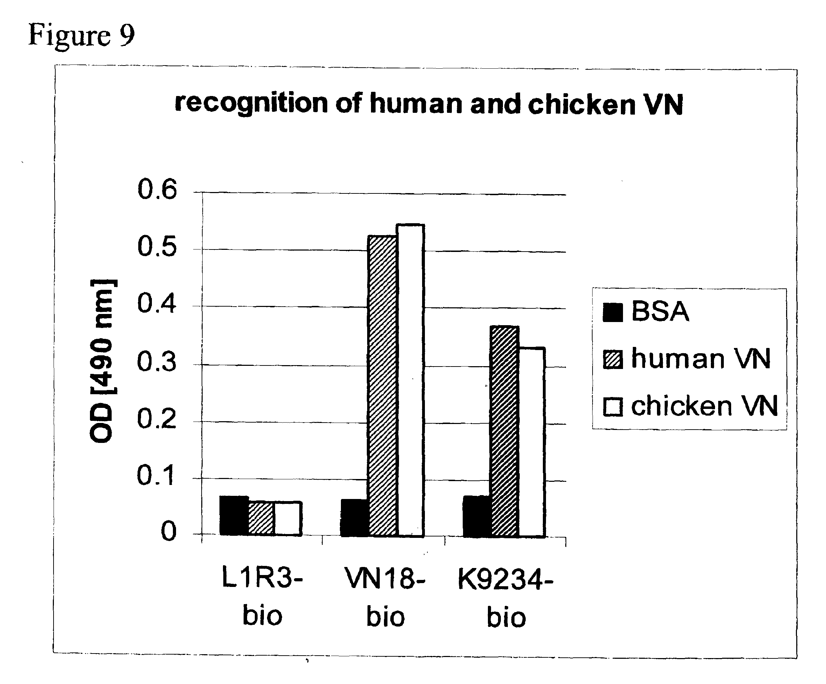

To determine whether Vn18 was also able to recognize vitronectin from other species, vitronectin was purified from human and chicken serum according to the method of Yatohgo et al. (1988). An ELISA was performed in which human or chicken vitronectin (10 µg/ml) were coated onto a 96-wells plate (Costar), blocked with bovine serum albumin (BSA). Binding of biotinylated IgG of Vn18, a control (phage generated) IgG (L1R3) or a polyclonal antibody directed against vitronectin (K9234) was detected with streptavidin-HRP, followed by staining with a peroxidase substrate. Human and chicken vitronectin show homology only in the region 268-336 of the hemopexin II (H2) domain (Nagano et al. 1992), indicating that the epitope for Vn18 is located in this area. Figure 9 shows that Vn18 recognized chicken vitronectin.

### Example 10. Effect of VN18 on platelet adhesion and function.

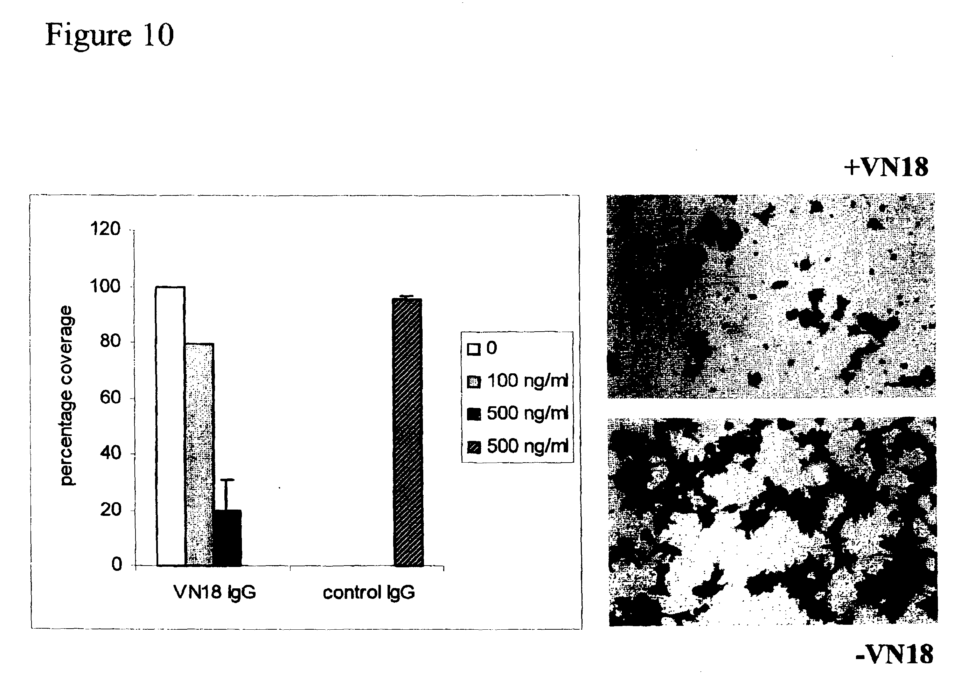

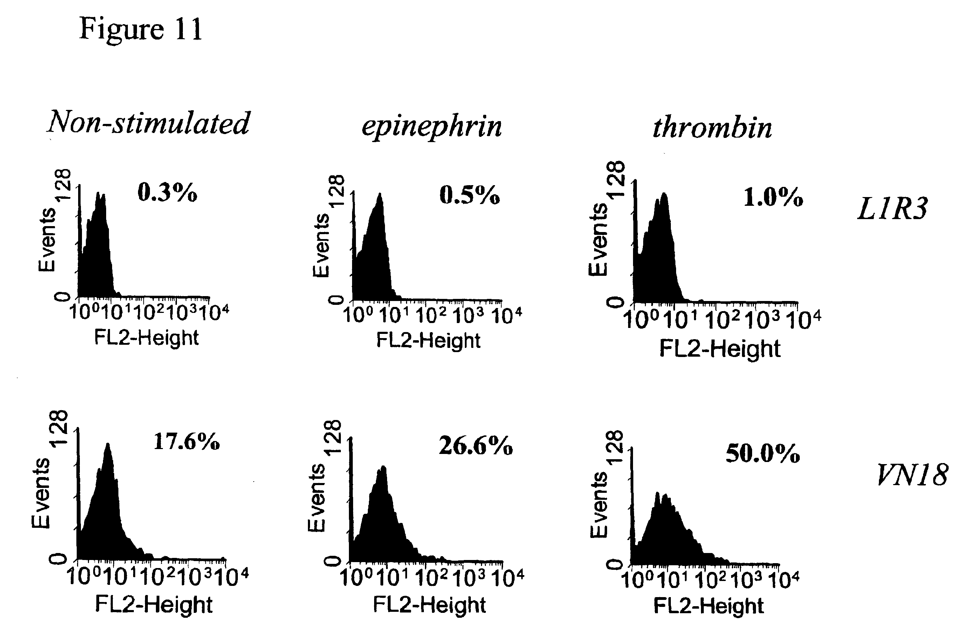

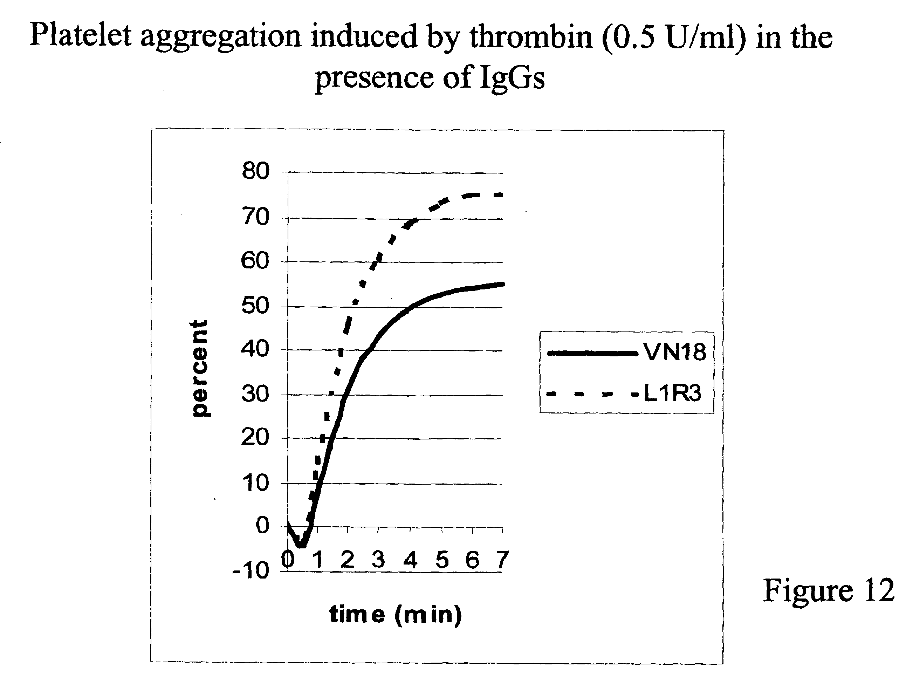

Activated vitronectin was sprayed on glass coverslips and the adhesion of isolated bloodplatelets was studied under flow. The isolated platelets were reconstituted in 4% human albumin solution (HAS) to which red blood cells were added, so no plasma proteins were present. Washed platelets (see below) were resuspended in Hepes-Tyrode buffer (10 mM Hepes, 0.145 mM NaCl, 5 mM KCL, 0.5 mM Na₂HPO₄, 1 mM MgSO₄, pH 7.25) to a concentration of 2x10⁵ platelets/µl. In the aggregation assay, 500 µl of this platelet suspension was pre-warmed to 37°C in the aggregometer for 4 min. Purified IgG of Vn18 or the control IgG L1R3 (see above) were pre-incubated for 1 min prior to addition of the stimulator thrombin (0.5 U/ml). Aggregation was monitored for 7 min under stirring. Figure 10 (left panel) shows that under these conditions Vn18 caused a concentration dependent inhibition on platelet adhesion, while the maximum concentration of the control antibody had no effect. In the absence of Vn18, some platelet aggregates were present. Figure 10 (right panel) shows that Vn18 addition inhibited mainly the formation of aggregates, suggesting that Vn18 is able to reduce the activation of platelets. Since this perfusion experiment was performed in the absence of exogenous vitronectin, Vn18 must react with vitronectin originating from the platelet intracellular pool. To investigate this, vitronectin expression on the platelet membrane was measured in the FACS after mild stimulation of the platelets with thrombin (0.5 U/ml) or epinephrin (100 nM). Platelet rich plasma (PRP) was obtained from citrated whole blood by centrifugation (10 min at 200xg, 20°C). One volume of Krebs-Ringer buffer (4 mM KCl, 107 mM NaCl, 20 mM NaHCO₃, 2 mM Na2₂SO₄, 4.76 mM citric acid, 14 mM Na-citrate and 5 mM d-glucose, pH 5.0) was added to one volume of PRP. The final pH was approximately 6. A platelet pellet was obtained by centrifugation (10 min at 500xg, 20°C), resuspended in Krebs-Ringer buffer (pH 6.0) and washed twice by centrifugation (10 min at 500xg, 20°C). After the second wash, platelets were resuspended in 4% human albumin solution (HAS) (4 mM KCl, 124 mM NaCl, 20 mM NaHCO₃, 2 mM Na₂ SO4, 2.5 mM CaCl₂, 1.5 mM MgCl₂, 5 mM d-glucose and 20 U/ml LMWH, pH 7.35) and incubated at 37°C in the absence or presence of epinephrin (100nM) or thrombin (0.5 U/ml). In the FACS analysis, 5x10⁵ /well of these platelets were stained using 10 µg/ml biotinylated VN18 or a control (phage display generated) IgG (L1R3), followed by streptavidin-PE. Figure 11 shows that non-stimulated platelets showed 17.6 % positive platelets, indicating that the washing procedure already caused some minor surface expression of platelet-vitronectin. After epinephrin stimulation, this amount increased up to 27%, whereas thrombin stimulation resulted in 47% VN-expressing platelets. A control antibody (which antibody?) showed no binding to the platelet membrane. To finally investigate the effect of Vn18 on platelet aggregation after mild stimulation of the platelets with thrombin (0.5 U/ml), another aggregation study was performed as described above. Figure 12 shows that in the presence of 100 µg/ml of Vn18, platelet aggregation was reduced with 20%, whereas a control antibody (L1R3) had no effect.

Although the invention has been described with reference to certain illustrative examples and preferred embodiments, the scope of the invention is to be determined by reference to the appended claims.

### BRIEF DESCRIPTION OF THE FIGURES

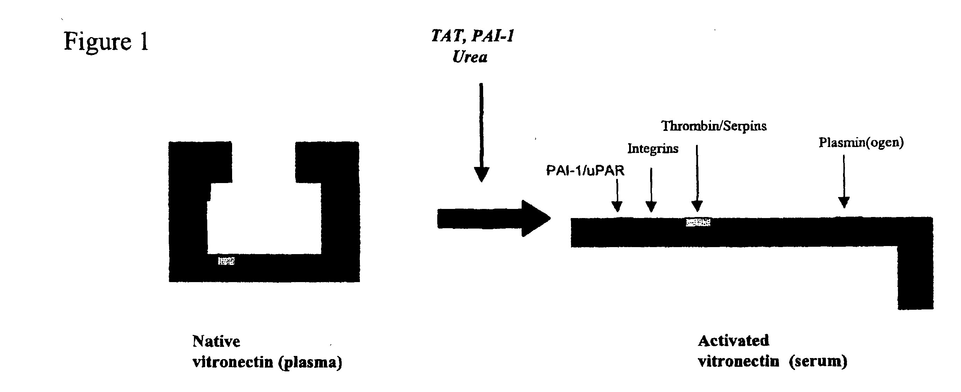

Figure 1. Schematic representation of the activation of vitronectin and its mediation by a complex of thrombin-antithrombin III that occurs during pathological processes, such as angiogenesis.

Figure 2. Specific detection of activated vitronectin by the scFv fragments Vn7, Vn18 and Vn26 in comparison to a rabbit polyclonal antiserum that recognizes both activated and native vitronectin.

Figure 3. A) Monoclonal phage antibody (mophabs) and B) scFv recognition of activated vitronectin in serum. Phage and scFv's were allowed to bind to coated urea-treated vitronectin in the absence or presence of plasma or serum. Binding of all mophabs and scFv's could be inhibited by serum, but not by plasma, strongly suggesting that the phages and scFv's also specifically recognized physiologically activated, serum vitronectin, but not native, plasma vitronectin

Figure 4. Immuno histochemistry illustrating the presence of activated vitronectin in carcinoma of the colon kidney, lung and other pathological specimens. Also shown are normal specimens of colon, kidney, lung and brorous tissue which indicate little activated vitronectin (see text).

Figure 5. A) Binding of endothelial cells to immobilised vitronectin analysed in the presence of various concentrations of anti-vitronectin antibody Vn18. B) Binding of endothelial cells to immobilised vitronectin analysed in the presence of three anti-vitronectin antibodies (Vn7, Vn18 and Vn26) in a concentration of 50 µg/ml.

Figure 6. Effect of anti-vitronectin antibody Vn18 on endothelial cell apoptosis. The number of apoptotic cells increased from 7.7% to 12.1%.

Figure 7. Binding of the indicated ligands on different sections of vitronectin (- no binding; + binding). The effect of Vn18 is expressed as percentage inhibition compared to a control IgG (smb against somatomedin B). Known binding epitopes are presented schematically (top drawing).

Figure 8. Immunoblot showing the in vitro interaction of Vn18 with a set of different purified fusion proteins (GST-vitronectin deletion mutants). S, C, H1 and H2 are the different domains in vitronectin (see text and Figure 7).

Figure 9. Interaction of Vn18 with chicken vitronectin.

Figure 10. Left panel. Effect of increasing concentrations of Vn18 antibody on platelet adhesion. Right panel. Formation of platelet aggregation in the presence (+Vn18) or in the absence (-Vn18) of the vitronectin specific IgG Vn18.

Figure 11. FACS analysis on Vn18 bound to platelets expressing vitronectin after stimulation by thrombin and epinephrin.

Figure 12. Effect of Vn18 on the formation of platelet aggregates after stimulation by thrombin.

### REFERENCES

Brooks PC, Clark RA, Cheresh DA. (1994a) Requirement of vascular integrin alpha v beta 3 for angiogenesis. Science 264:569-71
Brooks PC, Montgomery AM, Rosenfeld M, Reisfeld RA, Hu T, Klier G, Cheresh DA. (1994b) Integrin alpha v beta 3 antagonists promote tumor regression by inducing apoptosis of angiogenic blood vessels. Cell 79:1157-64
Brooks PC, Stromblad S, Klemke R, Visscher D, Sarkar FH, Cheresh DA. (1995) Antiintegrin alpha v beta 3 blocks human breast cancer growth and angiogenesis in human skin. J Clin Invest 96:1815-22
Carreiras F, Denoux Y, Staedel C, Lehmann M, Sichel F, Gauduchon P. (1996) Expression and localization of alpha v integrins and their ligand vitronectin in normal ovarian epithelium and in ovarian carcinoma. Gynecol Oncol 62:260
Carreiras F, Rigot V, Cruet S, Andre F, Gauduchon P, Marvaldi J. (1999a) Migration properties of the human ovarian adenocarcinoma cell line IGROV1: importance of alpha(v)beta3 integrins and vitronectin. Int J Cancer 80:285-94
Carreiras F, Cruet S, Staedel C, Sichel F, Gauduchon P. (1999b) Human ovarian adenocarcinoma cells synthesize vitronectin and use It to organize their adhesion. Gynecol Oncol 72:312-22
Dahlback K, Lofberg H, Dahlback B. (1988) Immunohistochemical studies on vitronectin in elastic tissue disorders, cutaneous amyloidosis, lichen ruber planus and porphyria. Acta Derm Venereol 68:107-15
De Kruif J, Boel E, Logtenberg T. (1995) Selection and application of human single chain Fv antibody fragments from a semi-synthetic phage antibody display library with designed CDR3 regions. J Mol Biol 248:97-105
De Kruif J, Storm G, van Bloois L, Logtenberg T. (1996) Biosynthetically lipid-modified human scFv fragments from phage display libraries as targeting molecules for immunoliposomes. FEBS Lett 399:232-6
Friedlander M, Brooks PC, Shaffer RW, Kincaid CM, Varner JA, Cheresh DA. (1995) Definition of two angiogenic pathways by distinct alpha v integrins. Science 270:1500
Gladson CL, Cheresh DA. (1991) Glioblastoma expression of vitronectin and the alpha v beta 3 integrin. Adhesion mechanism for transformed glial cells. J Clin Invest 88:1924-32
Graham and Karnovsky. (1966) The early stages of absorption of injected horseradish peroxidase in the proximal tubules of mouse kidney: ultrastructural cytochemistry by a new technique. 14:291-302
Guettier C, Hinglais N, Bruneval P, Kazatchkine M, Bariety J, Camilleri JP. (1989) Immunohistochemical localization of S protein/vitronectin in human atherosclerotic versus arteriosclerotic arteries. Virchows Arch A Pathol Anat Histopathol 414:309-13
Hayman EG, Pierschbacher MD, Ohgren Y, Ruoslahti E. (1983) Serum spreading factor (vitronectin) is present at the cell surface and in tissues. Proc Natl Acad Sci USA 80:4003
Huls GA, Heijnen IA, Cuomo ME, Koningsberger JC, Wiegman L, Boel E, Van der Vuurst de Vries AR, Loyson SA, Helfrich W, Berge Henegouwen GP, Van Meijer M, De Kruif J, Logtenberg T (1999) A recombinant, fully human monoclonal antibody with antitumor activity constructed from phage-displayed antibody fragments. Nat Biotechnol 17:276-281
Ignar DM, Andrews JL, Witherspoon SM, Leray JD, Clay WC, Kilpatrick K, Onori J, Kost T, Emerson DL. (1998) Inhibition of establishment of primary and micrometastatic tumors by a urokinase plasminogen activator receptor antagonist. Clin Exp Metastasis 16:9-20
Isik FF, Gibran NS, Jang YC, Sandell L, Schwartz SM. (1998) Vitronectin decreases microvascular endothelial cell apoptosis. J Cell Physiol 175:149-55
Jang YC, Arumugam S, Ferguson M, Gibran NS, Isik FF. (1998) Changes in matrix composition during the growth and regression of human hemangiomas. J Surg Res 80:9-15
Jankun J, Keck RW, Skrzypczak-Jankun E, Swiercz R. (1997) Inhibitors of urokinase reduce size of prostate cancer xenografts in severe combined immunodeficient mice. Cancer Res 57:559-63
Jaskiewicz K, Chasen MR, Robson SC. (1993) Differential expression of extracellular matrix proteins and integrins in hepatocellular carcinoma and chronic liver disease. Anticancer Res 13:2229-37
Kobayashi H, Gotoh J, Shinohara H, Moniwa N, Terao T (1994) Inhibition of the metastasis of Lewis lung carcinoma by antibody against urokinase-type plasminogen activator in the experimental and spontaneous metastasis model. Thromb Haemost 71:474-80
Köhler G and Milstein C. (1975) Continuous cultures of fused cells secreting antibody of predefined specificity. Nature 256:495
Kost C, Stuber W, Ehrlich HJ, Pannekoek H, Preissner KT. (1992) Mapping of binding sites for heparin, plasminogen activator inhibitor- 1, and plasminogen to vitronectin's heparin-binding region reveals a novel vitronectin-dependent feedback mechanism for the control of plasmin formation. J Biol Chem 267:12098
Landegren U (1984) Measurement of cell numbers by means of the endogenous enzyme hexosaminidase. Applications to detection of lymphokines and cell surface antigens. J Immunol Methods 67: 379-388
Liotta LA, Steeg PS, Stetler-Stevenson WG. (1991) Cancer metastasis and angiogenesis: an imbalance of positive and negative regulation. Cell 64:327-36
Loridon-Rosa B, Vielh P, Cuadrado C, Burtin P. (1988) Comparative distribution of fibronectin and vitronectin in human breast and colon carcinomas. An immunofluorescence study. Am J Clin Pathol 90:7-16
Min HY, Doyle LV, Vitt CR, Zandonella CL, Stratton-Thomas JR, Shuman MA, Rosenberg S. (1996) Urokinase receptor antagonists inhibit angiogenesis and primary tumor growth in syngeneic mice. Cancer Res 56:2428-33
Nagano Y, Hamano T, Nakashima N, Ishikawa M, Miyazaki K and Hayashi M (1992) Yolk vitronectin. Purification and differences from its blood homologue in molecular size, heparin binding, collagen binding, and bound carbohydrate J Biol Chem 267:24863-70
Pinedo HM, Verheul HM, D'Amato RJ, Folkman J. (1998) Involvement of platelets in tumour angiogenesis? Lancet 352:1775-7
Preissner KT, Zwicker L, Muller-Berghaus G. (1987) Formation, characterization and detection of a ternary complex between S protein, thrombin and antithrombin III in serum. Biochem J 243:105-11
Preissner KT, May AE, Wohn KD, Germer M, Kanse SM. (1997) Molecular crosstalk between adhesion receptors and proteolytic cascades in vascular remodelling. Thromb Haemost 78:88-95
Preissner KT, Seiffert D. (1998) Role of vitronectin and its receptors in haemostasis and vascular remodeling. Thromb Res 89:1-21
Reilly JT, Nash JR. (1988) Vitronectin (serum spreading factor): its localisation in normal and fibrotic tissue. J Clin Pathol 41:1269-72
Rosenblum G, Carsons S. (1996) Quantitation and distribution of vitronectin in synovial fluid and tissue of patients with rheumatic disease. Clin Exp Rheumatol 14:31-6
Schvartz I, Seger D, Shaltiel S. (1999) Vitronectin. Int J Biochem Cell Biol 31:539-44
Seiffert D, Schleef RR. (1996) Two functionally distinct pools of vitronectin (Vn) in the blood circulation: identification of a heparin-binding competent population of Vn within platelet alpha-granules. Blood 88:552-60
Seiffert D. (1997) Constitutive and regulated expression of vitronectin. Histol Histopathol 12:787-97
Sobel RA, Chen M, Maeda A, Hinojoza JR. (1995) Vitronectin and integrin vitronectin receptor localization in multiple sclerosis lesions. J Neuropathol Exp Neurol 54:202-13
Sumida H, Nakamura H, Satow Y. (1990) Distribution of vitronectin in the embryonic chick heart during endocardial cell migration. Arch Histol Cytol 53:81-8
Sumiyoshi K, Baba S, Sakaguchi S, Urano T, Takada Y, Takada A. (1991) Increase in levels of plasminogen activator and type-1 plasminogen activator inhibitor in human breast cancer: possible roles in tumor progression and metastasis. Thromb Res 63:59-71
Tomasini-Johansson BR, Sundberg C, Lindmark G, Gailit JO, Rubin K. (1994) Vitronectin in colorectal adenocarcinoma-synthesis by stromal cells in culture. Exp Cell Res 214:303-12
Tomasini-Johansson BR, Milbrink J, Pejler G. (1998) Vitronectin expression in rheumatoid arthritic synovia--inhibition of plasmin generation by vitronectin produced in vitro. Br J Rheumatol 37:620-9
Tsuchiya H, Katsuo S, Matsuda E, Sunayama C, Tomita K, Ueda Y, Binder BR. (1995) The antibody to plasminogen activator inhibitor-1 suppresses pulmonary metastases of human fibrosarcoma in athymic mice. Gen Diagn Pathol 141:41-8
Uhm JH, Gladson CL, Rao JS. (1999) The role of integrins in the malignant phenotype of gliomas. Front Biosci 4:D188-D199
Waltz DA, Natkin LR, Fujita RM, Wei Y, Chapman HA. (1997) Plasmin and plasminogen activator inhibitor type 1 promote cellular motility by regulating the interaction between the urokinase receptor and vitronectin. J Clin Invest 100:58-67
Weller M, Wiedemann P, Bresgen M, Heimann K. (1991) Vitronectin and proliferative intraocular disorders. I. A colocalisation study of the serum spreading factor, vitronectin, and fibronectin in traction membranes from patients with proliferative vitreoretinopathy. Int Ophthalmol 15:93-101
Werb Z. (1997) ECM and cell surface proteolysis: regulating cellular ecology. Cell 91:439-42
Yatohgo T, Izumi M, Kashiwagi H, Hayashi M. (1988) Novel purification of vitronectin from human plasma by heparin affinity chromatography. Cell Struct Funct 13:281-92
Yasumitsu H, Seo N, Misugi E, Morita H, Miyazaki K, Umeda M. (1993) Vitronectin secretion by hepatic and non-hepatic human cancer cells. In Vitro Cell Dev Biol Anim 29A:403-7
Yoneda A, Ogawa H, Kojima K, and Matsumoto I (1998) Characterization of the ligand binding activities of vitronectin: interaction of vitronectin with lipids and identification of the binding domains for various ligands using recombinant domains. Biochemistry 37:6351-60

## Claims

1. An antibody fragment comprising at least a CDR3 domain of a heavy chain of an antibody which has a higher affinity for activated vitronectin than for native vitronectin.

2. An antibody fragment according to claim 1 obtainable by subtractive phage selections on urea-activated vitronectin in the presence of plasma as a source of native vitronectin.

3. An antibody fragment according to claim 1 or 2, having a V-region comprising an amino acid sequence of SEQ. I.D. NO. 1, SEQ. I.D. NO. 2, SEQ. I.D. NO. 3 , SEQ. I.D. NO. 4 or a functional part, derivative and/or analogue thereof.

4. An antibody fragment, comprising at least 60 percent homology to an amino acid sequence of SEQ. I.D. NO. 1, SEQ. I.D. NO. 2, SEQ. I.D. NO. 3 , SEQ. I.D. NO. 4 or a functional part, derivative and/or analogue thereof.

5. An antibody fragment, comprising at least 95 percent homology to an amino acid sequence of SEQ. I.D. NO. 1, SEQ. I.D. NO. 2, SEQ. I.D. NO. 3 , SEQ. I.D. NO. 4 or a functional part, derivative and/or analogue thereof.

6. An antibody fragment according to anyone of claims 1-5, wherein said vitronectin is human vitronectin.

7. An antibody comprising an antibody fragment of anyone of claims 1-6.

8. An antibody according to claim 7, which is an IgG₁ antibody.

9. A pharmaceutical composition comprising an antibody fragment according to anyone of claims 1-6, and/or an antibody according to claim 7 or 8.

10. A method of diagnosing a disease state in a subject, said method comprising detecting for the presence of activated vitronectin in a biological sample taken from said subject.

11. The method according to claim 10, wherein the activated vitronectin is detected with use of an antibody and/or an antibody fragment.

12. The method according to claim 11, wherein said antibody comprises an antibody according to claims 7 or 8, and/or wherein said antibody fragment comprises a fragment according to anyone of claims 1-6.

13. The method according to claim 11 or 12, wherein said detection of activated vitronectin comprises detecting the presence of said antibody, antibody fragment, or a part thereof, bound to said vitronectin.

14. An apparatus for detecting the presence of activated vitronectin, derived by selection from a library using a phage display method.

15. A method of detecting the presence of activated vitronectin comprising using an antibody fragment according to anyone of claims 1-6, or an antibody of claim 7 or 8.

16. Use of an antibody fragment according to anyone of claims 1-6, or an antibody of claim 7 or 8, for the detection of the presence of activated vitronectin.

17. A use of an activated form of vitronectin as a target for antibody-mediated anti-vascular cancer therapy.

18. A method of cancer therapy comprising administering anti-activated vitronectin antibody fragments according to anyone of claims 1-6, and/or antibodies according to claim 7 or 8, which interfere with the adhesion and/or migration of tumor-endothelial cells, to a patient.

19. A method of manufacturing an antibody or an antibody fragment directed toward detecting the presence of activated vitronectin.

20. An apparatus for detecting the presence of activated vitronectin, using an antibody or an antibody fragment, said antibody or fragment derived by selection from a library comprising urea-treated vitronectin capable of binding to an antibody or antibody fragment of interest, such binding forming a receptor complex and thereby causing a change in a property associated with the binding partner; and a detection means for detecting the measurable change.

21. The apparatus of claim 20, wherein the receptor complex comprises an antibody or an antibody-binding fragment thereof derived by selection from a library using a phage display method.

22. An isolated peptide fragment comprising at least a CDR3 domain of a heavy chain of an antibody which has a higher affinity for activated vitronectin than for native vitronectin.

23. A peptide fragment according to claim 22 obtainable by subtractive phage selections on urea-activated vitronectin in the presence of plasma as a source of native vitronectin.

24. Use of an antibody according to claim 7 or 8, or an antibody fragment according to anyone of claims 1-6, for at least in part changing an adhesion property of activated vitronectin.

25. A use according to claim 24, wherein said adhesion property comprises the capability to bind to endothelial cells, extracellular matrix proteins, and/or platelets.

26. A use according to claim 25, wherein said changing of the capability to bind to extracellular matrix proteins prevents angiogenesis.

27. A method for at least in part inhibiting adhesion of an endothelial cell to activated vitronectin, comprising providing said vitronectin with an antibody fragment according to anyone of claims 1-6, an antibody according to claim 7 or 8, or a peptide according to claim 22 or 23.

28. A method for at least in part inducing cell death of an endothelial cell, associated with an extracellular matrix comprising activated vitronectin, comprising providing said matrix with an antibody fragment according to anyone of claims 1-6, an antibody according to claim 7 or 8, or a peptide according to claim 22 or 23.

29. A method according to claim 28, wherein said cell death comprises apoptosis.

30. A method for at least in part decreasing an interaction of activated vitronectin with collagen and/or endothelial cells, comprising providing said vitronectin with an antibody fragment according to anyone of claims 1-6, an antibody according to claim 7 or 8, or a peptide according to claim 22 or 23.

31. An isolated peptide comprising a vitronectin epitope which is recognized by an antibody fragment according to anyone of claims 1-6, an antibody according to claim 7 or 8, or a peptide according to claim 22 or 23.

32. A peptide according to claim 31, which comprises at least part of the vitronectin H2 domain.

33. An isolated or recombinant nucleic acid molecule encoding an antibody fragment according to anyone of claims 1-6, an antibody according to claim 7 or 8, or a peptide according to claim 22 or 23, or a functional part, derivative and/or analogue thereof.

34. An isolated or recombinant nucleic acid molecule encoding a peptide comprising a vitronectin epitope which is recognized by an antibody fragment according to anyone of claims 1-6, an antibody according to claim 7 or 8, or a peptide according to claim 22 or 23, or a functional part, derivative and/or analogue thereof.

35. A nucleic acid molecule according to claim 34, wherein said epitope comprises at least part of the vitronectin H2 domain.

36. A fusion protein comprising a peptide encoded by a nucleic acid molecule according to anyone of claims 33-35.

37. A method for at least in part inhibiting platelet aggregation in the presence of vitronectin, comprising providing said vitronectin with an antibody fragment according to anyone of claims 1-6, an antibody according to claim 7 or 8, or a peptide according to claim 22 or 23.

38. Use of an antibody fragment according to anyone of claims 1-6, an antibody according to claim 7 or 8, or a peptide according to claim 22 or 23 for the preparation of a medicament.

39. Use of an antibody fragment according to anyone of claims 1-6, an antibody according to claim 7 or 8, or a peptide according to claim 22 or 23 for the preparation of a medicament for at least in part inhibiting platelet aggregation.

40. Use of an antibody fragment according to anyone of claims 1-6, an antibody according to claim 7 or 8, or a peptide according to claim 22 or 23 for the preparation of a medicament for at least in part decreasing an interaction of activated vitronectin with collagen and/or endothelial cells.

41. Use of an antibody fragment according to anyone of claims 1-6, an antibody according to claim 7 or 8, or a peptide according to claim 22 or 23 for the preparation of a medicament for at least in part changing the development of blood vessels.

42. A method for at least in part changing the development of blood vessels, comprising influencing the remodeling process of the extracellular matrix of an endothelial cell.

43. A method according to claim 42, comprising providing said extracellular matrix with an antibody fragment according to anyone of claims 1-6, an antibody according to claim 7 or 8, or a peptide according to claim 22 or 23.

44. An antibody or functional part, derivative and/or analogue thereof, capable of at least in part inhibiting binding of an antibody fragment according to anyone of claims 1-6, an antibody according to claim 7 or 8, or a peptide according to claim 22 or 23, to activated vitronectin.


SEQUENCE LISTING

<110> Ubisys
<120> Activated Vitronectin as a Marker of Angiogenesis
      Detected with Phage Antibodies
<130> 2183-005
<140> filed concurrently
<141> Today's date
<160> 4
<170> PatentIn Ver. 2.1
<210> 1
<211> 15
<212> PRT
<213> Vitroncetin peptide
<400> 1
Cys Ala Arg Ala Arg Arg Leu Arg Leu Phe Asp Tyr Trp Gly Gln
 1               5                   10                  15
<210> 2
<211> 17
<212> PRT
<213> Vitroncetin peptide
<400> 2
Cys Ala Arg Asp Pro Arg Gly Pro Arg Ser Trp Leu Asp Tyr Trp
Gly Gln
 1               5                   10                  15

<210> 3
<211> 15
<212> PRT
<213> Vitroncetin peptide
<400> 3
Cys Ala Arg Asp Asp Arg Pro Arg Glu Leu Asp Ser Trp Gly Gln
 1               5                   10                  15
<210> 4
<211> 15

<212> PRT
<213> Vitroncetin peptide
<400> 4
Cys Ala Arg Ala Asn Ala Arg Ala Ala Phe Asp Ser Trp Gly Gln
1               5                   10                  15